# EUROPEAN PATENT SPECIFICATION

(11) **EP 1 401 362 B1**
(45) Date of publication and mention of the grant of the patent: **11.10.2006**
(21) Application number: 02769505.5
(22) Date of filing: 13.05.2002
(51) Int. Cl.: A61F 13/02

(54) **A WOUND DRESSING**
WUNDVERBAND
PANSEMENT

(30) Priority: 14.05.2001 GB 0111755
(43) Date of publication of application: 31.03.2004
(73) Proprietor: JOHNSON & JOHNSON MEDICAL LIMITED., Edinburgh EH2 4NH (GB)
(72) Inventor: EWER, Christopher John, Addingham, Ilkley, Yorkshire LS29 0SH (GB)
(74) Representative: James, Anthony Christopher W.P.
(86) International application number: PCT/GB2002/002202
(87) International publication number: WO 2002/091964

(56) References cited:
- DE-U- 8 430 484
- US-A- 4 499 896
- US-A- 4 666 441
- US-A- 4 990 144
- US-A- 5 486 158
- US-B1- 6 221 384

## Description

### Field of the invention

The present invention relates to wound dressings and in particular to absorbent wound dressings that can be cut to a desired size or shape prior to application to a wound.

### Background of the invention

A need exists for improved adhesive wound dressings that can be shaped by the care giver immediately before use so as to fit a given patient physique and/or wound size and configuration. This need is especially apparent in relation to sacral wound dressings.

Known sacral wound dressings have been of an 'island' type configuration having a backing layer, to a central region of which is fixed a smaller area of absorbent material. An adhesive border is usually provided on one side of the backing layer around the absorbent material for direct contact with a patient's skin. The adhesive border not only allows the dressing to be attached to a patient's skin around the wound, but it also acts as a barrier to foreign material such as dirt, water and moisture.

A problem with island type wound dressings is that if the adhesive layer or border becomes exposed to water, such as during patient bathing, the adhesive may become saturated and thereby loose its tackiness. Loss of tackiness may result in the dressing no longer being able to be held to the patient. Furthermore, there is limited scope for a care giver to cut the dressing down to a desired size immediately before use. If the dressing is cut prior to use, the width of the adhesive margin may be reduced to an undesirable extent. The adhesive margin could be removed completely in a part of the dressing, in which case absorbent material itself could be exposed to bath water and become saturated and the absorbent material will no longer absorb wound exudate. Island type dressings therefore have limited scope to vary from the size specification set by the wound dressing manufacturer.

EP-A-0 941 726 discloses an adhesive sacral wound dressing comprising a proximal wound contacting surface covered wholly or partly by a skin-friendly adhesive, a distal surface comprising a non-adhesive backing film, the backing film at least partly being covered by a cover layer wherein the cover layer comprises at least one incision line, and wherein the incision line stretches over the surface of the dressing leaving an uninterrupted zone at the edge of the dressing.

EP-A-0 768 071 discloses a sacral wound dressing in which a thin layer of a hydrocolloid-containing adhesive material is covered on one side by a flexible, transparent or semi-transparent backing film and, on the other side, by one or more removable release sheets. Linear depressions are provided along the film-backed side of the adhesive layer and are clearly visible through the backing film to serve as guide lines, flex lines, and/or hinge lines in the use of the dressing.

US-A-4499896 describes wound dressings having a reservoir compartment for retaining wound fluid. Figure 7 of US-A-4499896 shows an array of such dressings formed into a roll, and having an adhesive on a wound facing side thereof. Individual dressings comprising a single reservoir compartment are cut from the array before use.

US-A-4990144 describes wound dressings having a top sheet that has been formed with an array of recesses. The recesses are filled with a viscous pharmaceutical composition for topical application to the wound.

### Summary of the invention

It is an object of the invention to provide a wound dressing, in particular in dressing for the sacrum, which can be cut to a desired size for application to a patient's wound without degradation of the adhesive properties of the dressing. Other advantages of the invention will be apparent from the following description.

A first aspect of the invention resides in a wound dressing comprising: a backing sheet; a wound facing sheet having a wound facing surface and a back surface, said wound facing sheet being permeable to wound fluid over at least a first portion of its area and being attached to the backing sheet in a plurality of bonding regions; an array of compartments defined between the backing sheet and the wound facing sheet; an absorbent material contained within the compartments; and a liquid permeable adhesive layer provided on at least a portion of the wound facing surface of the wound facing sheet, characterised in that: the backing sheet and/or the wound facing sheet is profiled with an array of indentations corresponding to the location of the compartments, said compartments are arranged in a regular array that takes up at least 80% of the area of the dressing, and the area of each said compartment is from 25mm² to 400mm².

The purpose of the backing sheet is to provide a liquid- and microorganism-impermeable support to the dressing according to the invention. Preferably the backing sheet is semipermeable, that is to say it is substantially impermeable to water and microorganisms, but permeable to water vapour. The backing sheet preferably has a moisture vapour transmission rate (MVTR) of 300 to 5000 g/m²/24hrs and more preferably of 500 to 2000 g/m²/24hrs at 37.5 °C at 100% to 10% relative humidity difference. The backing sheet can have a thickness in the range of 10 to 1000 micrometers and more preferably 100 to 500 micrometers.

Suitable polymers for forming the backing sheet include polyurethanes and poly alkoxyalkyl acrylates and methacrylates such as those disclosed in GB-A-1280631. The backing sheet may comprises a continuous sheet of high density blocked polyurethane foam that is predominantly closed-cell. Suitable backing sheet materials are the polyurethane films available under the Registered Trade Mark ESTANE from B.F. Goodrich Company of Akron, Ohio.

The wound dressing also includes a wound facing sheet having a wound facing surface and a back surface. The wound facing sheet is liquid permeable over at least a first portion of its area so as to allow wound fluid to pass through readily in to the absorbent material, as described below. In certain preferred embodiments the liquid permeability of the wound facing sheet is directional. That is to say, the wound facing sheet allows fluid to pass through the wound facing sheet from the wound facing surface to the back surface, but blocks or restricts flow of the fluid back in the reverse direction (this reverse flow is also known as wet-back).

The wound facing sheet is preferably formed from a thermoplastic film-forming polymer. The polymer is conformable but preferably not substantially elastomeric. Suitable polymers include, but are not limited to, polyethylene, polypropylene, polyester, polyamides such as nylons, fluoropolymers such as polyvinylidene fluoride (PVDF) or polytetrafluoroethylene (PTFE), polyethylene vinyl acetate (EVA), and mixtures thereof. The wound facing sheet is preferably a polyolefin film.

Typically, the wound facing sheet has a thickness by weight (ASTM E252-84) of from 10 to 200 micrometers, and more preferably from 25 to 100 micrometers.

The wound facing sheet is typically formed from a substantially liquid-impermeable sheet material provided with small apertures in at least the said first portion thereof to allow the passage of aqueous liquids such as wound fluid. In certain preferred embodiments the apertures are in the form of directional capillaries, in which each capillary has a base substantially in the plane of the wound facing surface of the wound facing sheet and an apical opening remote from the wound facing surface on the back surface, in contact with an absorbent material. The capillaries may be tapered to provide rapid one-way wicking of fluid from the front of the wound facing sheet, with minimal wet-back. Wound facing sheets of this type are described in GB-A-1526778.

The tapered capillaries are typically substantially in the form of truncated cones and have a base opening dimension (the maximum opening dimension in the plane of the wound facing sheet of from 0.1 mm to 3 mm, and an apical opening dimension (remote from the plane of the wound facing sheet) of from 0.05 to 2 mm. The capillaries have a base opening dimension as defined typically of from 0.3 mm to 1 mm, and an apical opening dimension typically of from 0.1 to 0.5 mm.

The tapered capillaries typically have an average angle of taper (measured from the perpendicular to the plane of the wound facing sheet) of from 10 to 60 degrees. The embossed thickness of the wound facing sheet (by ASTM D374-79) is preferably from 0.2 to 2 mm, more preferably from 0.4 to 1 mm.

The density of the capillaries is typically from 10 to 400 per cm², and more preferably from 50 to 200 per cm². The open area of the wound facing sheet may be from 5 to 50% of the total area and more preferably from 10 to 25% of the total area.

In certain preferred embodiments, apertures allowing the passage of liquid are provided in the regions of the wound facing sheet over the absorbent compartments, but are substantially not present in regions of the wound facing sheet intermediate the absorbent compartments.

The wound facing sheet is attached directly or indirectly (i.e. through intermediate layers) to the backing sheet. Preferably the attachment is by a network of bonding regions, whereby the compartments for absorbent material are defined between the wound facing sheet and the backing sheet in the interstices of the bonding network. The network is an array of intersecting bonding lines, whereby there is provided the array of compartments between the wound facing sheet and the backing sheet. Preferably, the mean width of the bonding lines is less than 5 mm, more preferably less than 3 mm and most preferably less than 2 mm. The compartments are arranged in a regular array. Preferably, the compartments are arranged in a regular tesselation. Preferably, the compartments are substantially square, triangular, rectangular or hexagonal. The compartments take up at least about 80% of the area of the dressing, preferably at least about 90% of the area of the dressing. A high area fraction maximizes the compartment area available for the absorbent material, and minimises the area available for water ingress from the edges of the dressing. The ingress of water and other undesirables from the edges of the dressing may be further minimised by providing a hydrogel adhesive in the interstices between compartments on the wound facing side of the dressing as described further below.

The individual compartments of the dressing are preferably small in area relative to the area of the dressing. This is because, when the dressing is cut to size by the care giver immediately before use, the compartments at the edge of the dressing will be cut open, allowing ingress of water and microbes from the edge of the dressing into those compartments. Clearly, the area of the dressing that is thereby degraded is minimised by having small compartments. The area of the compartments is from 25 mm² to 400 mm², and preferably from 100 mm² to 250 mm². Preferably, the dressing comprises from about 10 to about 400 compartments, and more preferably from about 25 to about 200 compartments. It will be appreciated from the following description that the dressing may be manufactured in lengths or rolls comprising many more compartments.

The backing sheet and/or the wound facing sheet is profiled. That is to say, the sheet is not planar but comprises an array of indentations corresponding to the location of the compartments in the dressing. This enables relatively deep compartments to be provided with a relatively inelastic backing sheet and wound facing sheet. Preferably, the indentations are from 1 to 10 mm deep. The shape and distribution of the indentations corresponds to the shape and distribution of the compartments as described above.

In cases where the indentations are provided in the wound facing sheet, the dressing may comprise features intended to reduce ingress of water from (or escape of wound fluid to) the edges of the dressing. One such measure is to provide tightly packed indentations, preferably separated by no more than 1 mm. Another such measure is to fill the gaps between the indentations on the wound facing side with a hydrogel or other material that limits liquid flow between the indentations. Alternatively the wound facing sheet may be substantially flat, with the indentations provided in the backing sheet.

An absorbent material is provided within the absorbent compartments. Preferably, substantially all of the compartments contain the absorbent material. The absorbent material may comprise any of the absorbent materials conventionally used in the wound dressing art, including woven or nonwoven fabrics of hydrophilic fibers, hydrogels, superabsorbents, hydrophilic foams and sponges, and combinations thereof. In preferred embodiments the absorbent material includes an absorbent foam, such as an open celled hydrophilic polyurethane foam prepared in accordance with EP-A-0541391. Preferably, the dry uncompressed thickness of the absorbent layer is from about 1 to about 10 mm, preferably from about 2 to about 6 mm.

The absorbent layer may additionally comprise one or more active therapeutic or antimicrobial agents. Suitable therapeutic agents include growth factors, analgesics, local anaesthetics and steroids. Suitable antimicrobial agents include antiseptics such as silver compounds and chlorhexidine, and antibiotics. The therapeutic or antimicrobial agents are usually added in an amount of from 0.01 % to 5% by weight, based on the dry weight of the absorbent material.

A liquid permeable adhesive layer is provided on the wound facing surface of the wound facing sheet. The adhesive layer may comprise an interrupted layer of a conventional medically acceptable adhesive such as a polyurethane pressure-sensitive adhesive. Preferably, the adhesive is a hydrogel adhesive. That is to say, a composition that forms a sticky gel when hydrated. The hydrogel layer advantageously enables a moist wound environment to be maintained for prolonged periods, over a wide range of wound exudation rates. The wound facing sheet wicks away wound fluid to prevent excessive moisture in the wound, substantially without removal of the hydrogel or blocking of the wound facing sheet by the hydrogel. When the rate of wound exudate production falls, the hydrogel absorbs moisture vapour from the absorbent of the absorbent pockets and preserves a moist wound contacting surface. However, the hydrogel does not give rise to substantially increased wet-back through the wound facing sheet.

The term "hydrogel layer" refers generally to layers that interact with the wound surface under physiological conditions to maintain an elevated moisture level at the wound surface. The hydrogel layer forms a gel with water under physiological conditions of temperature and pH. Such hydrogel layers can be formed by the inclusion of medically acceptable macromolecular materials that have the ability to swell and absorb fluid while maintaining a strong integral structure. The hydrogel composition forms a gel that is substantially insoluble in water under physiological conditions, whereby the hydrogel is not washed away by the wound fluid. The hydrogel may be a biopolymer, and/or it may be bioabsorbable. That is to say, it may undergo gradual resorption *in vivo.*

Exemplary insoluble gels include certain cross-linked polyacrylate gels, calcium alginate gels, cross-linked hyaluronate gels, wherein the hydrogel sheet comprises a hydrogel material selected from gels formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof. Preferably, the gel adheres strongly to the surface of the wound facing sheet material to resist washing off by wound fluid. In certain embodiments the gel may be chemically bonded to the surface of the wound facing sheet.

The hydrogel layer preferably comprises a hydrogel material selected from polyurethane gels, biopolymer gels, carboxymethyl cellulose gels, hydroxyethyl cellulose gels, hydroxy propyl methyl cellulose, modified acrylamide and mixtures thereof. Suitable biopolymer gels include alginates, pectins, galactomannans, chitosan, gelatin, hyaluronates and mixtures thereof. Some of these biopolymer materials also promote wound healing.

The gels may be cross-linked, and the cross-linking may be either covalent or ionic.

The hydrogel adhesive may further comprise from 5 to 50% by weight on a dry weight basis of one or more humectants such as glycerol

In certain embodiments the hydrogel adhesive comprises a hydrogel material of the kind described in WO00/07638.

Alternatively or additionally to the gel-forming macromolecules, the hydrogel adhesive may comprise one or more emollients. Emollients are used to smooth the surface of skin and to increase the degree of hydration. They act either by occluding water loss from the outer sheet of the skin, or by improving water binding to the skin. Emollients are particularly useful in the treatment of awound facingic eczemas and ichthyoses. Preferred emollients include White Soft Paraffin, Yellow Soft Paraffin, Liquid paraffin, Urea Creams, Lanolin, Sodium Pyrrolidone Carboxylate (PCA Na), Evening primrose extract (gamma linolenic acid), Soya Oil, Tea Tree Oil, Coconut Oil, Almond Oil, Camomile Extract, Cod Liver Oil, Peanut Oil, Emu Oil, Aloe Vera, Sunflower oil, Avocado Oil, Jojoba Oil, Cocoamide, and mixtures thereof.

The hydrogel adhesive may additionally comprise one or more active therapeutic or antimicrobial agents. Suitable therapeutic agents include growth factors, analgesics, local anaesthetics and steroids. Suitable antimicrobial agents include antiseptics such as silver compounds and chlorhexidine, and antibiotics. The therapeutic or antimicrobial agents are usually added in an amount of from 0.01% to 5% by weight, based on the dry weight of the hydrogel material.

The hydrogel adhesive layer may be continuous or discontinuous, but in any case is preferably apertured in register with the apertures in the wound facing sheet so as not to obstruct passage of fluid into the capillaries even when the hydrogel is fully swelled. In other words, there is preferably substantially no hydrogel initially present in or covering the capillaries of the wound facing sheet. The hydrogel sheet may be applied by spraying (as described below) or by a printing or transfer process.

At least one protective cover sheet is preferably applied over the hydrogel sheet for protectively covering the wound dressing. The cover sheet is removed prior to application of the wound dressing to a wound. The cover sheet may be formed from release-coated paper or film. In certain embodiments there may be three release-coated paper cover sheets substantially as described in EP-A-0117632 to assist application of the relatively large sacral dressing. The wound dressing is preferably sterile and packaged in a microorganism-impermeable container.

In a second aspect, the invention resides in a method of manufacturing a wound dressing in accordance with the first aspect of the invention, comprising the steps of:
(a) providing a wound facing sheet having a back surface and a wound facing surface;
(b) providing a backing sheet;
(c) forming an array of indentations in the back surface of said wound facing sheet and/or in the backing sheet;
(d) providing an absorbent material within the indentations;
(e) attaching the backing sheet to the wound facing sheet along an array of bonding lines defined between said array of indentations, thereby entrapping the absorbent material within the indentations; and
(f) providing a liquid permeable adhesive layer on the wound facing surface of the wound facing sheet.

Preferred features of the process are as defined above in relation to preferred features of the product.

The array of indentations may be formed for example by embossing or thermoforming.

The method of manufacturing the wound dressing may further comprise the step of creating liquid permeable apertures in the wound facing sheet in at least the region of the compartments. This may be done, for example, by vacuum perforation either before or during the thermoforming step.

The process can advantageously be carried out continuously by providing the wound facing sheet and the backing sheet on respective rolls and feeding them continuously to a molding and bonding process.

In certain preferred embodiments the step of providing an absorbent material within the compartments comprises injecting a fluid foaming composition comprising a polyurethane prepolymer and water into the indentations and allowing the composition to foam and set to form a hydrophilic foam absorbent material. Preferably the foaming composition is substantially as described in EP-A-0541391.

### Brief description of the drawings

Notwithstanding any other forms which may fall within the scope of the present invention, specific embodiments of the invention will now be described, by way of example only, with reference to the accompanying drawings, in which:
Fig. 1 shows a top plan view of a first embodiment of a wound dressing showing the outer surface of the backing sheet, partially cut away;
Fig. 2 illustrates a cross-sectional view of the wound dressings of Fig. 1 along the line A-A;
Fig. 2A is an enlarged view of a part of the cross-sectional view shown in Fig. 2; and
Fig. 3A to Fig. 3D schematically illustrates the stages of an embodiment of a method of manufacturing a wound dressing according to the present invention.

### Detailed description of the embodiments

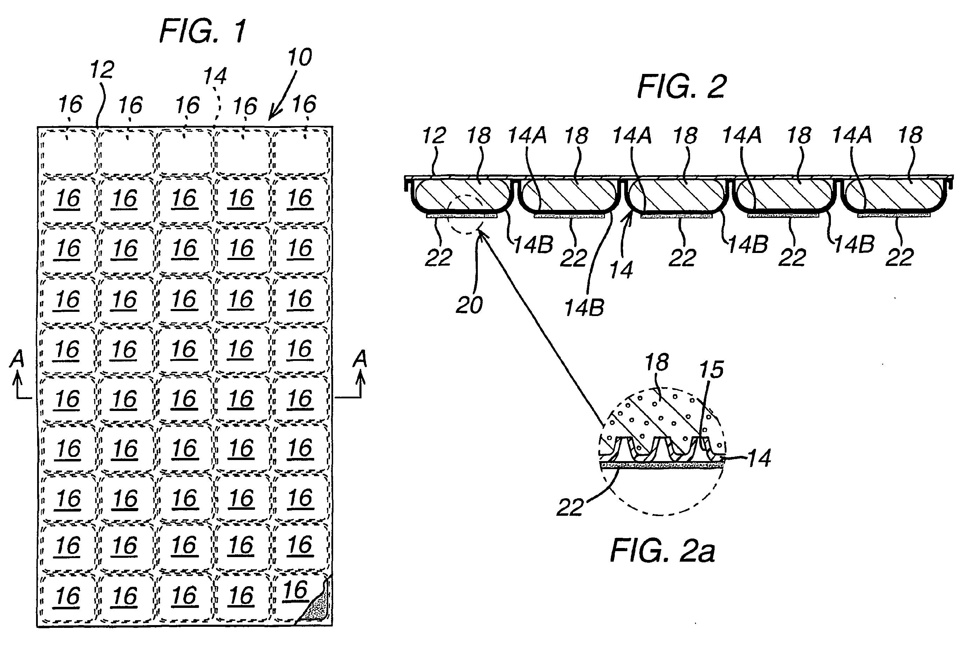

Referring to Fig. 1, the wound dressing 10 includes a backing sheet 12, which is semi-permeable in that it is permeable to water vapour, but not permeable to liquid water or wound exudate. Furthermore, the backing sheet 12 is also microorganism-impermeable. The backing sheet is formed from a microporous polyurethane of the kind conventionally used for island-type dressings.

Profiled wound facing sheet 14 is attached to the backing sheet 12 as will be described in further detail below. The wound facing sheet 14 is profiled to provide an array of indentations defining compartments 16 between the wound facing sheet 14 and the backing sheet 12.

The absorbent compartments 16 are substantially square in plan and have an area of about 100 mm². A small lateral gap exists between each of the adjacent absorbent pockets 16. The small lateral gap helps to minimize the lateral egress and ingress of water through the dressing. In practice, the lateral gaps fill with hydrogel, whereby very little lateral flow of liquid takes place.

Referring to Fig.2, it can be seen that the absorbent pockets 16 have absorbent material 18 enclosed therein. The absorbent material 18 is located between the backing sheet 12 and the wound facing sheet 14. The absorbent material 18 is an open-celled hydrophilic polyurethane foam of the kind described in EP-A-0541391.

Referring now to Fig. 2A, the detailed view of the wound contacting surface shown therein comprises the layer of hydrogel 22 extending substantially continuously over a perforated region of the top sheet 14. The hydrogel could be perforated in register with the top sheet perforations, but this is not necessary provided that the hydrogel is sufficiently water permeable.

The top sheet 14 is perforated with a plurality of tapered perforations 15 that provide rapid wicking of liquids into the absorbent foam layer 18, but tend to resist the flow of liquid back from the foam layer 18 to the wound surface. The size, shape and distribution of the perforations 15 are substantially as described in GB-A-1526778.

The hydrogel coating 22 acts as an adhesive when the wound dressing 10 is applied to a patient's wound.

Each capillary has a base substantially in the plane of the wound facing surface of the wound facing sheet and an apical opening remote from the wound facing surface on the back surface, in contact with an absorbent material. The conical capillaries provide rapid one-way wicking of fluid from the front of the wound facing sheet, with minimal wet-back. It will be appreciated that the capillaries could be provided only in regions of the wound facing sheet immediately covering the absorbent compartments.

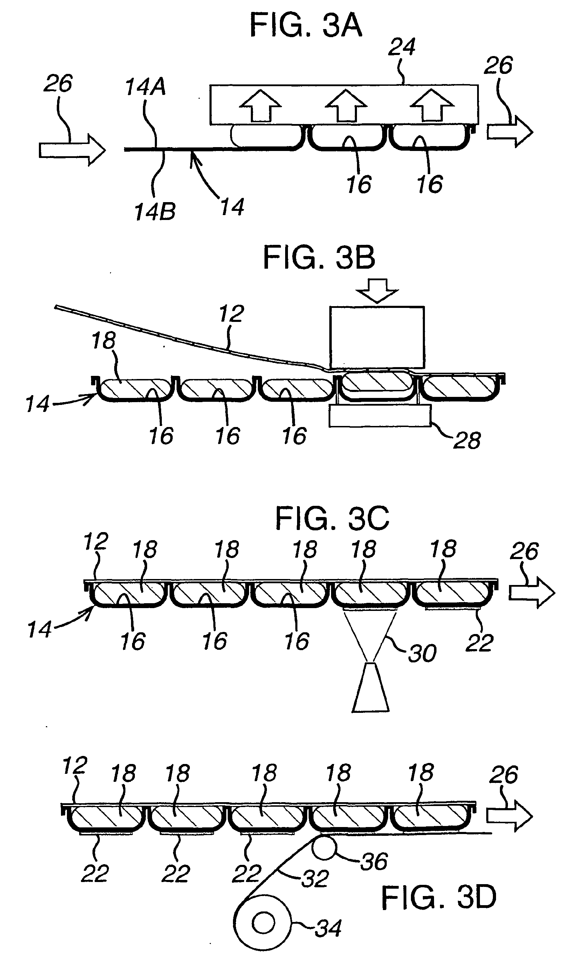

A cover sheet 32 (refer to Fig. 3D) is applied to the hydrogel sheet 22 for protectively covering the wound dressing 10. The cover sheet is removed prior to application of the wound dressing 10 to a wound. The cover sheet 22 comprises three release-coated paper cover sheets substantially as described in EP-A-0117632. The wound dressing is sterile and packaged in a microorganism-impermeable container.

To apply the wound dressing 10 to a patient's wound, the dressing is removed from the container and the dressing may then be cut to a desired size and shape by the care giver. The cover sheets are removed and the wound dressing 10 is applied substantially as described in EP-A-0117632.

When the wound dressing 10 is cut, some absorbent pockets 16 along the line of cutting will no longer contain a seal to external materials, such as water. However, the tackiness of the wound dressing as a whole does not degrade because the remaining absorbent compartments 16, including those adjacent to the cut absorbent compartments 16, maintain a seal between the patient's and the wound facing sheet 14. The adjacently disposed absorbent compartments 16 and the hydrogel form a protective seal to prevent the ingress of moisture to the dressing. Moisture ingress from external sources will affect only the very edge of the wound dressing 10. Thus, unlike 'island' type dressings of the prior art, the non-island type wound dressing 10 can be cut to any desired size for application to a patient's wound without substantial degradation of the adhesive or absorbent properties of the dressing.

A method of manufacturing the wound dressing 10 will now be described with reference to Fig. 3A to Fig. 3D.

Referring to Fig. 3A, there is shown a portion of the wound facing sheet 14 being placed under a vacuum plate 24. The vacuum plate 24 generates a vacuum above the wound facing sheet 14 to form indentations 16 by thermoforming (refer to Fig 2). The wound facing sheet 14 is fed from a roll (not shown) to the vacuum plate by a drive mechanism (not shown), in the direction of arrows 26.

During the thermoforming of the indentations 16, the tapered capillaries 15 described above may also be vacuum formed in the wound facing sheet 14 material to render it liquid permeable.

Referring now to Fig. 3B, once the indentations 16 have been formed with the capillaries, a layer of hydrophilic polyurethane foam 18 is dispensed into the indentations 16 in the wound facing sheet 14, so that the hydrophilic foam 18 is received into the indentations 16. The foam may be dispersed in solid pieces, or it may be dispensed by injecting a fluid foaming prepolymer mixture into the indentations and allowing the form to form, cure and dry.

As the hydrophilic foam 18 is inserted into the indentations 16, the backing sheet 12, which is fed from a feed roller, is placed over the sheet of hydrophilic foam 18. Consequently, the backing sheet 12 is sealed to the back surface 14A of the wound facing sheet 14 by heat and pressure sealing treatment being applied by a die 28. This ensures that the hydrophilic foam 18 is encapsulated within the absorption compartments 16. The sheets 12, 14 are then removed from the die 28.

Referring now to Fig. 3C, the back and wound facing sheets 12, 14 travel past a nozzle 30 which emits a spray of hydrogel to the wound facing surface 14B of the wound facing sheet 14, forming hydrogel sheet 22. It is preferable that the hydrogel is sprayed to the wound facing surface of each pocket 16 and not to between the gaps of the adjacent pockets 16. This may be achieved, for example, by spraying through a mask. The hydrogel layer is then dried.

Finally, as shown by Fig. 3D, a release membrane in the form of removable cover sheet 32 is then drawn off roller 34, past roller 36. The roller 36 urges the cover sheet 32 onto the hydrogel sheet 22 to provide a protective cover to the hydrogel before use of the dressing 10.

The roll 34 and the rollers used for feeding the back sheet 12 and the wound facing sheet 14, allow the final product dressing 10 to be manufactured in a continuous operation.

After the dressing has been formed, it can be cut to required shape by using an X, Y cutting device and a length of the wound dressing can be dispensed from a roll in use. A user typically removes a desired length of the wound dressing 12 from the roll according to the size of the patient's wound.

Once the rolled final wound dressing 10 product has been formed, it is placed immediately into a packaging device, such as a pick and place device.

It will be appreciated that the wound dressing 10 can be cut to any size or shape, allowing flexibility in the usage of the wound dressing. Therefore, it is no longer necessary to use dressings having a defined size and shape, resulting in a reduction in the wastage of the wound dressing.

Other embodiments of the invention may involve formation of indentations 16 in the backing sheet 12 rather than forming the pockets first in the wound facing sheet 14. Alternatively, the wound facing surface 14B of the wound facing sheet 14 and that back sheet 12 could be formed without indentations with the array of absorption pockets 16 residing between the back sheet 12 and wound facing sheet 14.

Additionally, the stages of manufacturing the above described embodiment are less than for island-type dressings, which also reduces the costs of manufacture. For example, there is no slitting or converting of backings, no requirement of foam slitting and no requirement for manual assembly as with island type dressings.

It would be appreciated by a person skilled in the art that numerous variations and/or modifications may be made to the present invention as shown in the specific embodiments without departipg from the scope of the invention as claimed. The present embodiments are therefore, to be considered in all respects to be illustrative and not restrictive.

## Claims

1. A wound dressing comprising:
a backing sheet (12);
a wound facing sheet (14) having a wound facing surface (14B) and a back surface (14A), said wound facing sheet (14) being permeable to wound fluid over at least a first portion of its area and being attached to the backing sheet (12) in a plurality of bonding regions;
an array of compartments (16) defined between the backing sheet (12) and the wound facing sheet (14);
an absorbent material (18) contained within the compartments (16); and
a liquid permeable adhesive layer (22) provided on at least a portion of the wound facing surface (14B) of the wound facing sheet (14), **characterised in that**:
the backing sheet and/or the wound facing sheet is profiled with an array of indentations corresponding to the location of the compartments (16), said compartments are arranged in a regular array that takes up at least 80% of the area of the dressing, and the area of each said compartment is from 25mm² to 400mm².

2. A wound dressing according to claim 1, wherein said backing sheet (12) is attached to the back surface of the wound facing sheet (14) by a network of bonding lines.

3. A wound dressing according to claim 1 or claim 2, wherein the adhesive layer (22) comprises a hydrogel.

4. A wound dressing according to any preceding claim, wherein said wound dressing further comprises at least one removable cover sheet on said adhesive.

5. A wound dressing according to any preceding claim, wherein said absorbent material (18) comprises a hydrophilic foam material.

6. A wound dressing according to any preceding claim, wherein the wound facing sheet (14) is adapted to allow moisture to travel in a forward direction from said wound toward said compartments (16) more readily than in a reverse direction opposite to said forward direction.

7. A wound dressing according to any preceding claim, wherein said compartments (16) are substantially square, triangular, rectangular or hexagonal.

8. A wound dressing according to any preceding claim, wherein the backing sheet (12) is heat-sealed to the back surface of the wound facing sheet (14).

9. A wound dressing according to any preceding claim, wherein the adhesive layer (22) comprises a hydrogel material selected from any one or more of the following: polyurethane gels, biopolymer gels, carboxymethyl cellulose gels, hydroxyethyl cellulose gels hydroxypropyl methyl cellulose, modified acrylamides and mixtures thereof.

10. A wound dressing according to any preceding claim, wherein the adhesive layer (22) comprises a hydrogel material selected from gels formed from selected from any one or more of the following: vinyl alcohols, vinyl esters, vinyl ethers and carboxy vinyl monomers, meth(acrylic) acid, acrylamide, N-vinyl pyrrolidone, acylamidopropane sulphonic acid, pluronic (block polyethylene glycol, block polypropylene glycol)polystyrene maleic acid, NN- dimethylacrylamide, diacetone acrylamide or acryloyl morpholine.

11. A wound dressing according to any preceding claim, wherein the adhesive layer (22) comprises an emollient.

12. A wound dressing according to claim 11, wherein the emollient is selected from the group consisting of white soft paraffin, yellow soft paraffin, liquid paraffin, urea creams, lanolin, sodium pyrrolidone carboxylate, evening primrose extract (gamma linolenic acid), soya oil, tea tree oil, coconut oil, almond oil, camomile extract, cod liver oil, peanut oil, emu oil, aloe vera, sunflower oil, avocado oil, jojoba oil, cocoamide, and mixtures thereof.

13. A wound dressing according to any preceding claim, wherein the adhesive layer (22) or the absorbent sheet (18) comprises an active therapeutic agent or an antimicrobial agent.

14. A wound dressing according to claim 13, wherein the adhesive layer (22) comprises a silver compound.

15. A wound dressing according to any preceding claim, wherein the dressing is sterile and packaged in a microorganism-impermeable container.

16. A method of manufacturing a wound dressing according to any of claims 1 to 15, comprising the steps of:
(a) providing a wound facing sheet (14) having a back surface and a wound facing surface;
(b) providing a backing sheet (12);
(c) forming an array of indentations on at least one of the back surface of the wound facing sheet (14) and the backing sheet (12);
(d) providing absorbent material (18) within the indentations;
(e) attaching the backing sheet (12) to the wound facing sheet (14) along a network of bonding lines defined between the array of indentations, thereby entrapping the absorbent material within the indentations; and
(f) providing a liquid permeable adhesive layer (22) on the wound facing surface of the wound facing sheet (14).

17. A method as claimed in claim 16, wherein the adhesive layer (22) is applied to the wound facing surface by a spray nozzle.

18. The method as claimed in claim 16 or 17, wherein the array of indentations is formed by heating the wound facing sheet (14) and/or the backing sheet (12) over a mould and subjecting it to a vacuum to take up the shape of the mould.

19. The method as claimed in any one of claims 16 to 18, further comprising the step of: vacuum perforating said wound facing sheet (14) to render it liquid permeable.

20. The method as claimed in any one of claims 16 to 19, wherein the back surface of the wound facing sheet (14) and backing sheet (12) are attached by heat and pressure sealing treatment.

21. The method as claimed in any one of claims 16 to 20, wherein: the wound facing sheet (14) is provided on a roll before being introduced to the mould;
wherein the backing sheet (12) is provided on another roll before being attached to the wound facing sheet (14); and
wherein during manufacturing of the wound dressing, the wound facing sheet (14) is fed from the roll and the backing sheet (12) is fed from said another roll to an assembly point where the assembly of the wound dressing is undertaken as a continuous process.

## Patentansprüche

1. Wundverband, welcher umfaßt:
einen Stützbogen (12);
einen der Wunde gegenüberliegenden Bogen (14) mit einer der Wunde gegenüberliegenden Oberfläche (14b) und einer Rückfläche (14a), wobei der der Wunde gegenüberliegende Bogen (14) gegenüber Wundfluid über wenigstens einen ersten Bereich seiner Fläche permeabel ist und an dem Stützbogen (12) in einer Vielzahl von Bindungsbereichen angefügt ist;
eine Anordnung von Fächern (16), die zwischen dem Stützbogen (12) und dem der Wunde gegenüberliegenden Bogen (14) definiert sind;
ein Absorbensmateial (18), das innerhalb der Fächer (16) enthalten ist; und
eine flüssigkeitspermeable Klebstoffschicht (22), die auf wenigstens einem Bereich der der Wunde gegenüberliegenden Oberfläche (14b) des der Wunde gegenüberliegenden Bogens (14) bereitgestellt ist, **dadurch gekennzeichnet, daß**: der Stützbogen und/oder der der Wunde gegenüberliegende Bogen mit einer Anordnung von Einkerbungen entsprechend der Lage der Fächer (16) profiliert ist, wobei die Fächer in einer regelmäßigen Anordnung angeordnet sind, die wenigstens 80% der Fläche des Verbandes einnimmt, und wobei die Fläche jedes Faches von 25 mm² bis 400 mm² ist.

2. Wundverband nach Anspruch 1, wobei der Stützbogen (12) an der Rückfläche des der Wunde gegenüberliegenden Bogens (14) durch ein Netzwerk von Bindungslinien angefügt ist.

3. Wundverband nach Anspruch 1 oder Anspruch 2, wobei die Klebstoffschicht (22) ein Hydrogel umfaßt.

4. Wundverband nach einem der vorangehenden Ansprüche, wobei der Wundverband ferner wenigstens einen entfernbaren Deckbogen auf dem Klebstoff umfaßt.

5. Wundverband nach einem der vorangehenden Ansprüche, wobei das Absorbensmaterial (18) ein hydrophiles Schaummaterial umfaßt.

6. Wundverband nach einem der vorangehenden Ansprüche, wobei der der Wunde gegenüberliegende Bogen (14) angepaßt ist, um es Feuchtigkeit zu ermöglichen, sich in einer Vorwärtsrichtung von der Wunde in Richtung auf die Fächer (16) leichter als in einer Rückrichtung entgegensetzt zur Vorwärtsrichtung zu bewegen.

7. Wundverband nach einem der vorangehenden Ansprüche, wobei die Fächer (16) im wesentlichen quadratisch, dreieckig, rechteckig oder hexagonal sind.

8. Wundverband nach einem der vorangehenden Ansprüche, wobei der Stützbogen (12) an die Rückfläche des der Wunde gegenüberliegenden Bogens (14) mittels Wärme gesiegelt ist.

9. Wundverband nach einem der vorangehenden Ansprüche, wobei die Klebstoffschicht (22) ein Hydrogelmaterial umfaßt, das ausgewählt ist aus einem oder mehreren der folgenden: Polyurethangelen, Biopolymergelen, Carboxymethylcellulosegelen, Hydroxyethylcellulosegelen, Hydroxypropylmethylcellulose, modifizierten Acrylamiden und Mischungen derselben.

10. Wundverband nach einem der vorangehenden Ansprüche, wobei die Klebstoffschicht (22) ein Hydrogelmaterial umfaßt, das aus gebildeten Gelen ausgewählt ist, die ausgewählt sind aus einem oder mehreren der folgenden: Vinylalkoholen, Vinylester, Vinylether und Carboxyvinylmonomeren, Meth(acryl)säure, Acrylamid, N-Vinylpyrrolidon, Acylamidopropansulfonsäure, pluorinische (Blockpolyethylenglykol, Blockpolypropylenglykol)polystyrolmaleinsäure, NN-Dimethylacrylamid, Diacetonacrylamid oder Acryloylmorpholin.

11. Wundverband nach einem der vorangehenden Ansprüche, wobei die Klebstoffschicht (22) einen Weichmacher umfaßt.

12. Wundverband nach Anspruch 11, wobei der Weichmacher ausgewählt ist aus der Gruppe bestehend aus weißem Weichparaffin, gelbem Weichparaffin, flüssigem Paraffin, Harnstoffcremes, Lanolin, Natriumpyrrolidoncarboxylat, ausgleichendem Primoseextrakt (Gamma-Linolensäure), Sojaöl, Teebaumöl, Kokosnußöl, Mandelöl, Kamillenextrakt, Lebertranöl, Erdnußöl, Emuöl, Aloe Vera, Sonnenblumenöl, Avocadoöl, Jojobaöl, Cocoamid und Mischungen derselben.

13. Wundverband nach einem der vorangehenden Ansprüche, wobei die Klebstoffschicht (22) oder der Absorbensbogen (18) ein aktives therapeutisches Agens oder ein antimikrobielles Agens umfaßt.

14. Wundverband nach Anspruch 13, wobei die Klebstoffschicht (22) eine Silberverbindung umfaßt.

15. Wundverband nach einem der vorangehenden Ansprüche, wobei der Verband steril ist und in einem für Mikroorganismen impermeablen Behälter verpackt ist.

16. Verfahren zum Herstellen eines Wundverbands nach einem der Ansprüche 1 bis 15, welches die Schritte umfaßt:
(a) Bereitstellen eines der Wunde gegenüberliegenden Bogens (14) mit einer Rückfläche und einer der Wunde gegenüberliegenden Oberfläche;
(b) Bereitstellen eines Stützbogens (12);
(c) Bilden einer Anordnung von Einkerbungen auf wenigstens einer der Rückfläche des der Wunde gegenüberliegenden Bogens (14) und des Stützbogens (12);
(d) Bereitstellen von Absorbensmaterial (18) innerhalb der Einkerbungen;
(e) Anfügen des Stützbogens (12) an den der Wunde gegenüberliegenden Bogen (14) entlang eines Netzwerks von Bindungslinien, die zwischen der Anordnung von Einkerbungen definiert werden, wodurch das Absorbensmaterial innerhalb der Einkerbungen eingeschlossen wird; und
(f) Bereitstellen einer flüssigkeitspermeablen Klebstoffschicht (22) auf der der Wunde gegenüberliegenden Oberfläche des der Wunde gegenüberliegenden Bogens (14).

17. Verfahren nach Anspruch 16, wobei die Klebstoffschicht (22) auf die der Wunde gegenüberliegenden Oberfläche durch eine Sprühdüse aufgetragen wird.

18. Verfahren nach einem der Ansprüche 16 oder 17, wobei die Anordnung von Einkerbungen durch Erwärmen des der Wunde gegenüberliegenden Bogens (14) und/oder des Stützbogens (12) über einer Bildungsform und Unterziehen desselben einem Vakuum, um die Form der Bildungsform anzunehmen, gebildet wird.

19. Verfahren nach einem der Ansprüche 16 bis 18, weiter umfassend den Schritt: Vakuumperforieren des der Wunde gegenüberliegenden Bogens (14), um ihn flüssigkeitspermeabel zu machen.

20. Verfahren nach einem der Ansprüche 16 bis 19, wobei die Rückfläche des der Wunde gegenüberliegenden Bogens (14) und der Stützbogen (12) durch Wärme- und Druckversiegelungsbehandlung angefügt werden.

21. Verfahren nach einem der Ansprüche 16 bis 20, wobei: der der Wunde gegenüberliegende Bogen (14) auf einer Rolle bereitgestellt wird, bevor er in die Bildungsform eingeführt wird;
wobei der Stützbogen (12) auf einer weiteren Rolle bereitgestellt wird, bevor er an den der Wunde gegenüberliegenden Bogen (14) angefügt wird; und
wobei während der Herstellung des Wundverbandes der der Wunde gegenüberliegende Bogen (14) von der Rolle zugeführt wird und der Stützbogen (12) von der anderen Rolle zugeführt wird zu einem Montagepunkt, wo die Montage des Wundverbandes als ein kontinuierliches Verfahren ausgeführt wird.

## Revendications

1. Pansement, comprenant :
■ une feuille de support (12) ;
■ une feuille orientée vers la plaie (14) ayant une surface orientée vers la plaie (14B) et une surface arrière (14A), ladite feuille orientée vers la plaie (14) étant perméable aux fluides de la plaie sur au moins une première partie de sa superficie et étant fixée à la feuille de support (12) dans une pluralité de zones de liaison ;
■ une rangée de compartiments (16) définis entre la feuille de support (12) et la feuille orientée vers la plaie (14) ;
■ un matériau absorbant (18) contenu à l'intérieur des compartiments (16) ; et
■ une couche adhésive perméable aux liquides (22) apportée sur au moins une partie de la surface orientée vers la plaie (14B) de la feuille orientée vers la plaie (14), **caractérisé en ce que** :
■ la feuille de support et/ou la feuille orientée vers la plaie est profilée avec une rangée de dentelures correspondant à l'emplacement des compartiments (16), lesdits compartiments étant disposés en une rangée régulière qui occupe au moins 80 % de la superficie du pansement, et la superficie de chacun desdits compartiments allant de 25 mm² à 400 mm².

2. Pansement selon la revendication 1, dans lequel ladite feuille de support (12) est fixée à la surface arrière de la feuille orientée vers la plaie (14) par un réseau de lignes de liaison.

3. Pansement selon la revendication 1 ou 2, dans lequel la couche adhésive (22) comprend un hydrogel.

4. Pansement selon l'une quelconque des revendications précédentes, dans lequel ledit pansement comprend au moins une feuille de couverture amovible sur ledit adhésif.

5. Pansement selon l'une quelconque des revendications précédentes, dans lequel ledit matériau absorbant (18) comprend un matériau en mousse hydrophile.

6. Pansement selon l'une quelconque des revendications précédentes, dans lequel la feuille orientée vers la plaie (14) est adaptée pour permettre à l'humidité de circuler dans un sens dirigé vers l'avant à partir de ladite plaie jusqu'aux dits compartiments (16) plus facilement que dans un sens inverse au dit sens dirigé vers l'avant.

7. Pansement selon l'une quelconque des revendications précédentes, dans lequel lesdits compartiments (16) sont sensiblement carrés, triangulaires, rectangulaires ou hexagonaux.

8. Pansement selon l'une quelconque des revendications précédentes, dans lequel la feuille de support (12) est thermocollée à la surface arrière de la feuille orientée vers la plaie (14).

9. Pansement selon l'une quelconque des revendications précédentes, dans lequel la couche adhésive (22) comprend un matériau hydrogel sélectionné parmi un ou plusieurs des suivants : gels de polyuréthane, gels biopolymères, gels de carboxyméthylcellulose, gels de hydroxyéthylcellulose, hydroxypropylméthylcellulose, acrylamides modifiés et mélanges de ceux-ci.

10. Pansement selon l'une quelconque des revendications précédentes, dans lequel la couche adhésive (22) comprend un matériau hydrogel sélectionné parmi les gels formés à partir d'un ou plusieurs des composants suivants : alcools vinyliques, esters vinyliques, éthers vinyliques et monomères carboxyvinyliques, acide (méth)-acrylique, acrylamide, N-vinyle pyrrolidone, acide sulfonique d'acylamidopropane, polystyrène pluronique (glycol de polyéthylène à blocs, glycol de polypropylène à blocs) acide maléique, NN-diméthyl-acrylamide, diacétone-acrylamide ou morpholine d'acryoyle.

11. Pansement selon l'une quelconque des revendications précédentes, dans lequel la couche adhésive (22) comprend un émollient.

12. Pansement selon la revendication 11, dans lequel l'émollient est sélectionné dans le groupe comprenant la paraffine tendre blanche, la paraffine tendre jaune, l'huile de paraffine, les crèmes à base d'urée, la lanoline, la pyrrolidone carboxylate de sodium, l'huile d'onagre (acide gamma-linoléique), l'huile de soja, l'huile de Mélaleuca à feuilles alternes, l'huile de coco, l'huile d'amandes, l'extrait de camomille, l'huile de foie de morue, l'huile d'arachides, l'huile d'émeu, l'huile d'aloès, l'huile de tournesol, l'huile d'avocat, l'huile de jojoba, le cocoamide, et des mélanges de ceux-ci.

13. Pansement selon l'une quelconque des revendications précédentes, dans lequel la couche adhésive (22) ou la feuille absorbante (18) comprend un agent thérapeutique actif ou un agent antimicrobien.

14. Pansement selon la revendication 13, dans lequel la couche adhésive (22) comprend un mélange d'argent.

15. Pansement selon l'une quelconque des revendications précédentes, dans lequel le pansement est stérile et emballé dans récipient imperméable aux micro-organismes.

16. Méthode de fabrication d'un pansement selon l'une quelconque des revendications 1 à 15, comprenant les étapes consistant à :
(a) apporter une feuille orientée vers la plaie (14) ayant une surface arrière et une surface orientée vers la plaie ;
(b) apporter une feuille de support (12) ;
(c) former une rangée de dentelures sur au moins une surface arrière de la feuille orientée vers la plaie (14) et de la feuille de support (12) ;
(d) apporter du matériau absorbant (18) à l'intérieur des dentelures ;
(e) fixer la feuille de support (12) à la feuille orientée vers la plaie (14) le long d'un réseau de lignes de liaison définies entre la rangée de dentelures, enfermant ainsi le matériau absorbant à l'intérieur des dentelures ; et
(f) apporter une couche adhésive perméable aux liquides (22) sur la surface orientée vers la plaie de la feuille orientée vers la plaie (14).

17. Méthode selon la revendication 16, dans laquelle la couche adhésive (22) est appliquée sur la surface orientée vers la plaie par un bec pulvérisateur.

18. Méthode selon la revendication 16 ou 17, dans laquelle la rangée de dentelures est formée en chauffant la feuille orientée vers la plaie (14) et/ou la feuille de support (12) sur un moule et en la soumettant à une aspiration pour lui donner la forme du moule.

19. Méthode selon l'une quelconque des revendications 16 à 18, comprenant en outre l'étape consistant à perforer par aspiration ladite feuille orientée vers la plaie (14) afin de la rendre perméable aux liquides.

20. Méthode selon l'une quelconque des revendications 16 à 19, dans laquelle la surface arrière de la feuille orientée vers la plaie (14) et la feuille de support (12) sont fixées par un traitement de scellage à chaud et sous pression.

21. Méthode selon l'une quelconque des revendications 16 à 20, dans laquelle : la feuille orientée vers la plaie (14) est positionnée sur un rouleau avant d'être introduite dans le moule ;
dans laquelle la feuille de support (12) est positionnée sur un autre rouleau avant d'être fixée à la feuille orientée vers la plaie (14) ; et
dans laquelle au cours de la fabrication du pansement, la feuille orientée vers la plaie (14) est alimentée depuis le rouleau et la feuille de support (12) est alimentée depuis ledit autre rouleau vers un point d'assemblage où l'assemblage du pansement est entrepris en tant que procédé continu.
